# EUROPÄISCHE PATENTSCHRIFT

(11) **EP 1 348 445 B1**
(45) Veröffentlichungstag und Bekanntmachung des Hinweises auf die Patenterteilung: **10.04.2013**
(21) Anmeldenummer: 03003632.1
(22) Anmeldetag: 18.02.2003
(51) Int. Cl.: A61L 2/00

(54) **Verfahren zur Abtrennung von Viren aus einer Proteinlösung durch Nanofiltration**
Method for separating viruses from a protein solution by nanofiltration
Procédé de séparation de virus d'une suspension de protéines par nanofiltration

(30) Priorität: 15.03.2002 DE 10211632
(43) Veröffentlichungstag der Anmeldung: 01.10.2003
(73) Patentinhaber: CSL Behring GmbH, 35041 Marburg (DE)
(72) Erfinder: Lengsfeld, Thomas, 35041 Marburg (DE); Schneider, Heinrich, 35094 Lahntal (DE)

(56) Entgegenhaltungen:
- EP-A- 1 136 084
- EP-A- 1 153 608
- WO-A-00/29041
- WO-A-99/19343
- WO-A-99/23111
- WO-A-99/64441
- BURNOUF-RADOSEVICH M ET AL: "NANOFILTRATION, A NEW SPECIFIC VIRUS ELIMINATION METHOD APPLIED TO HIGH-PURITY FACTOR IX AND FACTOR XI CONCENTRATES", VOX SANGUINIS, S. KARGER AG, BASEL, CH, vol. 67, no. 2, 1 August 1994 (1994-08-01) , pages 132-138, XP000609860, ISSN: 0042-9007

## Beschreibung

Gegenstand der Erfindung ist die Nanofiltration von Proteinlösungen, mit der es gelingt, Viren praktisch vollständig abzutrennen.

Für kleine Proteinmoleküle ist die Nanofiltration ein sehr wirksames Verfahren zur Entfernung von Viren. Dabei muss die Porengröße des Filters kleiner als der effektive Durchmesser des zu entfernenden Virus sein. Außerdem sind die Temperatur, die Stoffeigenschaften und die Pufferbedingungen von entscheidender Bedeutung bei der Durchführung einer Nanofiltration. Frühere Studien haben bereits gezeigt, dass der Parvovirus durch Filter mit einem Porendurchmesser von 15 nm zuverlässig entfernt werden kann. Die Nanofiltration ist auch schon zur Abtrennung des Hepatitis-A-Virus und des Parvovirus aus Faktor IX-Präparaten eingesetzt worden, wobei sich Filter wie Viresolve 70, Planova 15 N und Pall Ultipor DV20 bewährt haben (M. Burnouf-Radosevich et al., Vox Sang 67_132-138_1994). Der Blutgerinnungsfaktor IX hat jedoch ein kleines Molekulargewicht von 56 kDa und wird deshalb nicht durch die für die Nanofiltration eingesetzten Membranen zurückgehalten. Große Proteine wie Fibrinogen, von-Willebrand-Faktor und Faktor VIII sind jedoch bisher als zu groß angesehen worden, um sie durch Filtration durch einen Nanofilter mit einer Knotengröße mit 15 bis 35 nm von Viren zu befreien.

Fibrinogen ist ein 340 kDa hexameres (a₂b₂g₂) Glykoprotein. Die Kristallstruktur von natürlichem Hühner-Fibrinogen zeigt eine Länge von 46 nm. Elektronenmikroskopische Messungen haben gezeigt, dass Fibrinogen eine Dreiknotenstruktur mit einer Länge von 47,5 nm und einer Knotengröße von 6,5 nm aufweisen. Außerdem führt die Hydration zur Vergrößerung des Fibrinogenmoleküls. Deshalb konnten bisher nur Filtrationsverfahren für Fibrinogen bei einer Filterporengröße von 35 nm beschrieben werden Damit ist der Nachteil verbunden, dass bei einer Porengröße von 35 nm kleinere, nicht umhüllte Viren wie der Hepatits-A-Virus und der Parvovirus nicht entfernt werden können. Obwohl Nanofilter mit einer Porengröße von 20 und weniger nm zur Verfügung stehen, mit denen auch Hepatits-A-Viren oder Parvoviren entfernt werden könnten, kann deshalb von diesen Filtern bei der Reinigung von Fibrinogen bisher kein Gebrauch gemacht werden, da dieses Molekül für diese Porengröße als zu voluminös anzusehen ist (Roberts, P., Vox Sang, 1995; 69:82-83).

Da jedoch die Nanofiltration eine besonders schonende Methode zum Entfernen von Viren aus Proteinlösungen ist und dabei die biologische Aktivität der Proteine voll erhalten bleibt, stellte sich die Aufgabe, ein Verfahren zur Virusentfemung aus Proteinlösungen durch Nanofiltration zu entwickeln, mit dem auch großvolumige Proteinmoleküle einer Nanofiltration zugänglich gemacht werden können.

Gelöst wird diese Aufgabe durch ein Verfahren, bei dem der Proteinlösung vor der Nanofiltration zur Verminderung oder Verhinderung der Aggregation und der Ausbildung einer Hydrathülle der Proteinmoleküle eine chaotrope Substanz aus der Gruppe bestehend aus Arginin, Guanidin, Citrullin, Harnstoff oder ihren Derivaten zugesetzt wird und anschließend die Lösung durch einen Filter mit einer Porengröße zwischen 15 bis 25 nm filtriert wird.

Besonders geeignet ist das Verfahren zur Abtrennung von Viren aus einer Fibrinogenlösung oder aus einer Lösung eines Blutgerinnungsfaktors, zum Beispiel des Faktors VIII.

Das Verfahren kann bei Zimmertemperatur durchgeführt werden, wodurch eine thermische Belastung und der damit verbundene biologische Aktivitätsverlust der Proteinmoleküle vermieden wird. Proteine mit Molekulargewichten von 200 bis 340 kDa können so bei einer Porengröße von 15 bis 25 nm von Viren befreit werden. Insbesondere Mitglieder der Parvovirusfamilie, die eine globuläre Partikelgröße von 18 bis 26 nm besitzen und Hepatitis-A-Viren mit einer stabförmigen Struktur von 6 bis 17 nm Durchmesser und etwa 48 nm Länge können so entfernt werden.

Das erfindungsgemäße Filtrationsverfahren lässt sich darüber hinaus mit sehr guten Erfolg mit einem konventionellen Pasteurisierungsverfahren kombinieren, wodurch die Abreicherung von Viren noch weiter erhöht wird.

Die für das erfindungsgemäße Verfahren eingesetzten Nanofilter sind im Handel erhältlich und können z.B. unter den Bezeichnungen DV-15, DV-20 u.a. erworben werden.

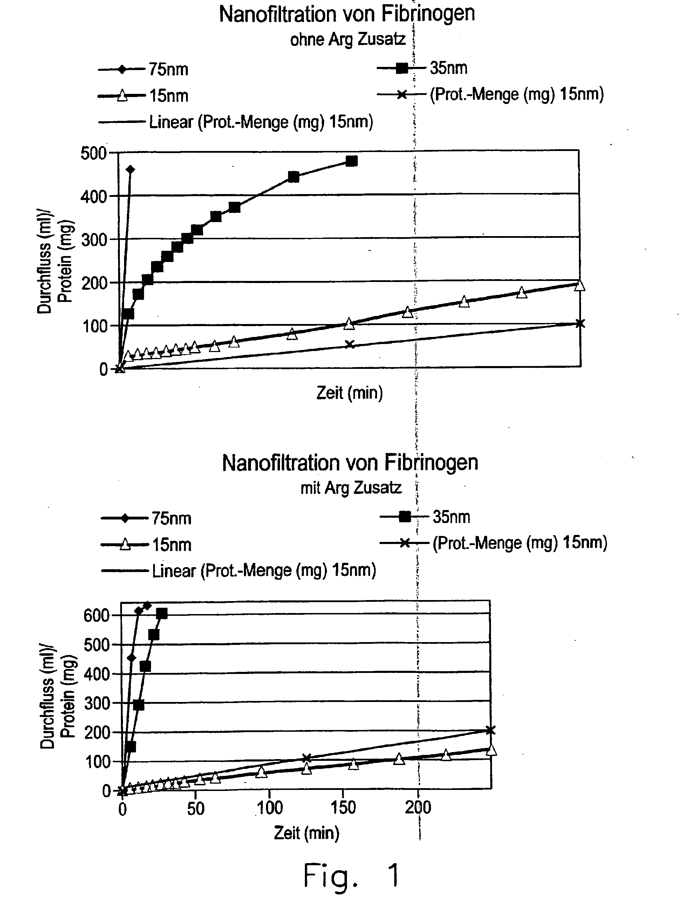

In der als Anlage beigefügten Fig. 1 wird gezeigt, wie die Menge der durch Nanofiltration gewonnenen, virenfreien Fibrinogenlösung von der Porengröße des Filters einerseits und vom Zusatz von Argininmonohydrochlorid andererseits abhängt. Es ist zu erkennen, dass bei Zusatz von Argininmonohydrochlorid auch schon bei einer Porengröße von 15 nm befriedigende Mengen von Fibrinogen-Filtrat gewonnen werden können.

## Patentansprüche

1. Verfahren zur Abtrennung von Viren aus einer Proteinlösung durch Nanofiltration, **dadurch gekennzeichnet, dass** man die Proteinlösung vor der Nanofiltration zur Verminderung oder Verhinderung der Aggregation der Proteinmoleküle mit einer chaotropen Substanz aus der Gruppe bestehend aus Arginin, Guanidin, Citrullin, Harnstoff oder ihren Derivaten versetzt und anschließend die Lösung durch ein Filter mit einer Porengröße zwischen 15 bis 25 nm gibt, wobei die Proteine ein Molekulargewicht von 200 bis 340 kDa aufweisen.

2. Verfahren nach Anspruch 1, **dadurch gekennzeichnet, dass** man eine Virusabtrennung in einer Fibrinogenlösung oder in einer Lösung eines Blutgerinnungsfaktors durchführt.

3. Verfahren nach den Ansprüchen 1 und 2, **dadurch gekennzeichnet, dass** man die Nanofiltration bei Zimmertemperatur durchführt.

4. Verfahren nach den Ansprüchen 1 bis 3, **dadurch gekennzeichnet, dass** man die Proteinlösung zusätzlich zur Nanofiltration einer Pasteurisierung unterwirft.

## Claims

1. Method for separating off viruses from a protein solution by means of nanofiltration, **characterized in that** a chaotropic substance from the group consisting of arginine, guanidine, citrulline, urea or their derivatives is added to the protein solution, prior to the nanofiltration, in order to decrease or prevent aggregation of the protein molecules, and the solution is then passed through a filter having a pore size of between 15 and 25 nm, the proteins having a molecular weight of from 200 to 340 kDa.

2. Method according to Claim 1, **characterized in that** a virus separation is carried out in a fibrinogen solution or in a solution of a blood coagulation factor.

3. Method according to Claims 1 and 2, **characterized in that** the nanofiltration is carried out at room temperature.

4. Method according to Claims 1 to 3, **characterized in that**, in addition to the nanofiltration, the protein solution is subjected to a pasteurization.

## Revendications

1. Procédé pour séparer des virus d'une solution de protéines par nanofiltration, **caractérisé en ce que**, avant la nanofiltration, pour diminuer ou empêcher l'agrégation des molécules de protéines, on ajoute à la solution de protéines une substance chaotropique du groupe consistant en l'arginine, la guanine, la citrulline, l'urée ou leurs dérivés, puis on fait passer la solution à travers un filtre ayant une grosseur de pore comprise entre 15 et 25 nm, les protéines présentant une masse moléculaire de 200 à 340 kDa.

2. Procédé selon à revendication 1, **caractérisé en ce qu'**on met en oeuvre la séparation des virus dans une solution de fibrinogène ou dans une solution d'un facteur de coagulation du sang.

3. Procédé selon les revendications 1 et 2, **caractérisé en ce qu'**on met en oeuvre la nanofiltration à la température ambiante.

4. Procédé selon les revendications 1 à 3, **caractérisé en ce qu'**on soumet la solution de protéines, en plus de la nanofiltration, à une pasteurisation.
